Europäisches Patentamt

European Patent Office

Office européen des brevets

(11) Numéro de publication: **0 390 652**

**A1**

(12) **DEMANDE DE BREVET EUROPEEN**

(21) Numéro de dépôt: 90400789.5

(22) Date de dépôt: 22.03.90

(51) Int. Cl.5: **G03B 21/132, G03B 42/02, F21K 7/00**

(30) Priorité: 29.03.89 FR 8904087

(43) Date de publication de la demande:
03.10.90 Bulletin 90/40

(84) Etats contractants désignés:
**DE ES GB IT NL**

(71) Demandeur: **GENERAL ELECTRIC CGR S.A.**
**100, rue Camille-Desmoulins**
**F-92130 Issy les Moulineaux(FR)**

(72) Inventeur: **Romeas, René**
**Cabinet Ballot-Schmit, 7 rue le Sueur**

**F-75116 Paris(FR)**
Inventeur: **Pelissonnier, Bernard**
**Cabinet Ballot-Schmit, 7 rue le Sueur**
**F-75116 Paris(FR)**
Inventeur: **Gregoire, Yves**
**Cabinet Ballot-Schmit, 7 rue le Sueur**
**F-75116 Paris(FR)**

(74) Mandataire: **Schmit, Christian Norbert Marie**
**et al**
**Cabinet Ballot-Schmit 7, rue Le Sueur**
**F-75116 Paris(FR)**

(54) **Table lumineuse de radiographie.**

(57) On réalise une table lumineuse de radiographie en utilisant un écran graphique (20) couplé à un microprocesseur (23) et utilisé en luminosité inversée. Dans ces conditions, l'écran graphique émet une lumière en presque tous ces points sauf en des endroits ($I_{25}$) particuliers dont on peut contrôler la position aux moyens d'un boîtier de commande (24). On place au-dessus de l'écran graphique un cliché radiographique (6) à examiner. On peut utiliser, par l'intermédiaire du boîtier de commande, les régions non-éclairées de cet écran pour pointer des endroits particuliers de ce cliché. La table trouve plus particulièrement son utilisation dans l'étude des clichés stéréographiques de mammographie. Elle permet une détermination plus rapide et plus précise des régions avec lésion des seins à examiner.

FIG_2

## Table lumineuse de radiographie.

La présente invention a pour objet une table lumineuse de radiographie. Elle trouve son utilisation particulièrement dans le domaine médical, où elle sert à l'examen des clichés radiographiques. Elle peut néanmoins servir dans d'autres domaines. Dans un mode d'utilisation particulièrement intéressant, la table de l'invention sert pour l'examen des clichés stéréographiques : c'est à dire des clichés correspondant à des irradiations d'un corps à examiner sous deux angles différents. Ces clichés sont présentés simultanément sur la table lumineuse afin de favoriser pour l'observateur, ou pour une machine de reconnaissance, une visualisation en relief, ou la localisation dans l'espace d'endroits particuliers du corps soumis à cet examen.

On connaît dans l'état de la technique des tables lumineuses de radiographie. Celles-ci comportent essentiellement une dalle translucide placée au-dessus d'un dispositif d'éclairement. Les clichés radiographiques à examiner sont posés sur cette dalle. Les contours et les structures qu'ils représentent sont vus par la différence d'atténuation d'énergie lumineuse transmise, depuis le dispositif d'éclairement, par le cliché vers l'oeil de l'observateur.

Dans un examen stéréotaxique, dans le but de repérer des coordonnés XYZ, dans un espace à trois dimensions, d'une région particulière (en général une lésion) d'un corps examiné, on dépose sur une telle dalle deux clichés pris sous deux angles d'incidence différents. Les tables lumineuses destinées à cette reconnaissance comportent en outre des index ponctuels mobiles qui permettent de repérer avec précision des points à analyser sur les clichés. Ces index sont normalement mus par un dispositif mécanique actionné par un opérateur. Cet opérateur actionne ce dispositif mécanique pour les placer en coïncidence avec les points à analyser du film. Un dispositif de recopie enregistre les déplacements mécaniques effectués et envoie en correspondance les coordonnés de la position de l'index à un ordinateur. Cet ordinateur, d'une manière connue, peut procéder au traitement nécessaire pour, en utilisant les positions de deux index placés d'une manière homologue sur chacune des parties correspondantes du cliché stéréographique, calculer les coordonnés dans l'espace de la lésion. Ces coordonnés peuvent être affichées sur un moniteur de télévision ou sur tout autres moyens. Elles peuvent par ailleurs être utilisées dans un dispositif de traitement thérapeutique ou un dispositif de prélèvement de tissus en vue d'une analyse cytologique.

Les dispositifs de recopie comportent, pour la transmission de la position de l'index, une partie mécanique. Cette partie mécanique entraîne que ces dispositifs de recopie sont peu précis. En effet, par flexion ou par jeux il y a toujours une erreur de recopie de la position exacte de l'index. Par ailleurs, ces dispositifs de recopie sont d'une part onéreux, d'autre part lourds. Par leur caractère mécanique, ils sont enfin susceptibles de se dérégler et nécessitent en conséquence un entretien fréquent : nettoyage, graissage, recalibration. Un tel appareil est par exemple décrit dans FR-A-2 248 535.

Par ailleurs, pour l'examen de clichés stéréographiques, il peut être intéressant de disposer de plus d'un jeu de deux index homologues. Compte tenu du caractère mécanique du pointage des index, la mise en place d'un index peut parfois nécessiter le déplacement d'un index précédent parce que les mécanismes de pointage de ces index ne peuvent pas se chevaucher ou se croiser. Pour la même raison, le nombre de couples d'index homologues utilisable est faible : un maximum de trois est connu.

L'invention a pour objet de remédier à ces inconvénients en proposant une table lumineuse dont la dalle et le dispositif d'éclairement sous-jacent sont remplacés par un écran graphique tel qu'on en trouve aujourd'hui de nombreux modèles dans le commerce. Dans un exemple, on choisira un écran à matrice de diodes à électroluminescence. On pourrait utiliser également des écrans de technologies différentes : plasma, cristaux liquides, voire même un tube à rayons cathodiques.

L'utilisation de l'écran graphique est cependant différente de celle qu'on lui donne habituellement. En effet, un écran graphique comporte normalement un plan peu lumineux : seul l'endroit où doivent être matérialisés les traits d'un dessin apparaît lumineux. Dans l'invention, on agit différemment. L'écran est lumineux partout. Il éclaire suffisamment le cliché par en-dessous. Mais il reste sombre à l'endroit correspondant à celui où on veut pointer l'index. La brillance de cet écran doit donc être suffisante pour être du même ordre que dans l'état de la technique. Par ailleurs, l'écran, dont la surface externe sert de dalle, doit de préférence être plat. De cette manière il reçoit les films radiographiques sans les voiler.

Un écran graphique se caractérise essentiellement par son aptitude à permettre le repérage de ses éléments d'image, en luminosité inversée, des points ombrés (rendus sombres), par rapport à un repère associé. Ce repérage peut être obtenu par tous moyens. Par exemple pour un repérage dans le temps, dans un signal vidéo d'affichage sur l'écran, on détermine l'apparition d'une impulsion de ce signal correspondant à la partie ombrée. Dans le cas où on utilise une mémoire d'image, les adresses (les coordonnés) des points ombrés sur cet écran sont en relation directe avec les adresses des cellules mémoires de cette mémoire qui contiennent une information d'ombrage (par exemple un bit zéro) par rapport au reste de l'image lumineuse (correspondant à un bit un).

L'invention a donc pour objet une table lumineuse de radiographie, comportant une surface éclairante pour illuminer un cliché radiographique placé sur cette surface, et au moins un index mobile qu'on peut déplacer sur cette surface pour venir pointer un endroit particulier du cliché, caractérisée en ce qu'elle comporte un écran graphique contrôlé par un microprocesseur, pour dispenser une lumière éclairante en tous ses points, sauf là où on veut pointer l'index.

En utilisation de stéréographie, il y a plusieurs index. Les repérages des index sont couplés l'un à l'autre et le programme de traitement, de type connu, prélève directement les adresses de ces index pour déterminer la région de l'espace à trois dimensions où se trouve la partie correspondante à pointer.

L'invention sera mieux comprise à la lecture de la description qui suit et l'examen des figures qui l'accompagnent. Celles-ci ne sont données qu'à titre indicatif et nullement limitatif de l'invention.

Les figures montrent :

- figure 1 : la représentation schématique d'un mammographe servant à la réalisation d'un cliché stéréographique à l'examen duquel sert l'invention ;

- figure 2 : la représentation schématique des moyens essentiels de la table lumineuse de l'invention.

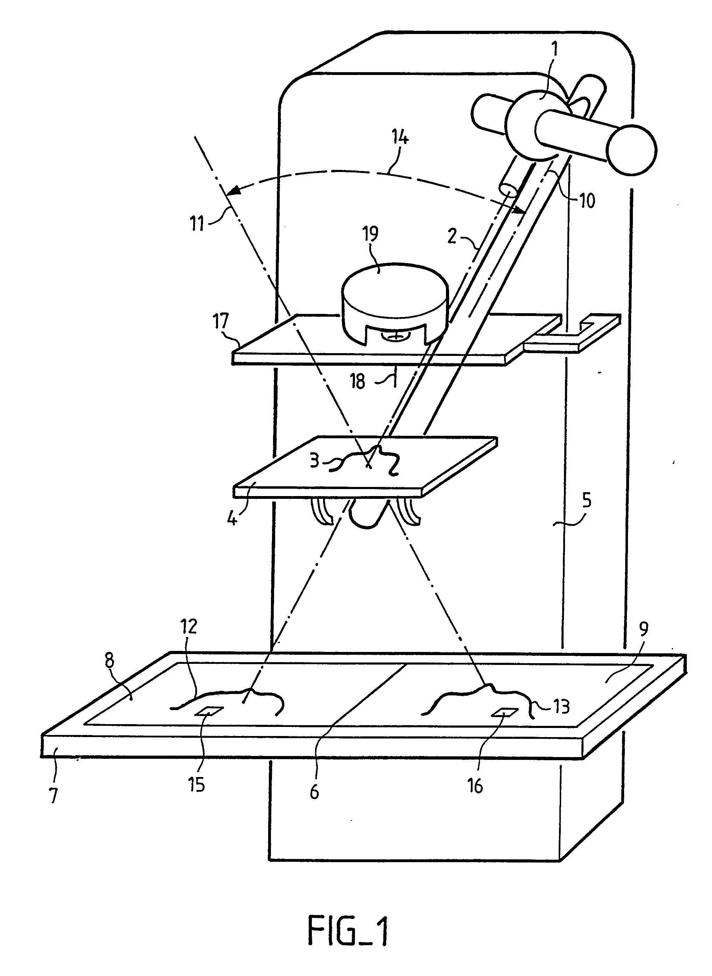

La figure 1 montre un mammographe utilisable dans le domaine médical pour la prise de clichés stéréographiques. Ce mammographe comporte comme tout appareil de radiologie un tube 1 à rayons X émettant un rayonnement X d'axe principal 2 en direction d'un sein 3 à radiographier porté sur un plateau porte-sein 4. Le plateau porte-sein 4 est fixé à un bâti 5 du mammographe. Le rayonnement X, après avoir irradié le sein 3 vient impressionner une plaque photosensible 6 placée dans une cassette 7. La cassette est maintenue en correspondance du tube 1 à rayons X. Un mammographe utilisable en stéréographie possède la particularité de pouvoir impressionner une même plaque 6 en différents endroits, par exemple dans une partie gauche 8 ou dans une partie droite 9, selon que le tube 1 à rayons X a respectivement pris une première orientation 10 ou une seconde orientation 11 par rapport au sein 3. Ces différentes orientations sont rendues possibles en faisant porter le tube 1 par un mât oscillant autour d'un centre de rotation. Les images du sein 3 sont alors projetées respectivement en 12 et 13 sur la plaque 6.

A partir des deux images 12 et 13 ainsi accolées de préférence sur un même cliché, et correspondant à une différence d'incidence d'angle 14, on sait, en pointant sur chacune de ces images des régions caractéristiques homologues, respectivement 15 et 16, retrouver avec un ordinateur la position dans l'espace (par exemple au-dessus du plateau porte-sein 4)d'un endroit du sein 3 dont les projections ont correspondu à ces images particulières. Dans ces conditions, il devient possible, à partir d'un plateau porte-aiguille 17, comportant une aiguille de biopsie 18, mue par un moteur 19, de venir piquer le sein 3 à un endroit particulier où on souhaite faire un prélèvement de tissus en vue de leur analyse.

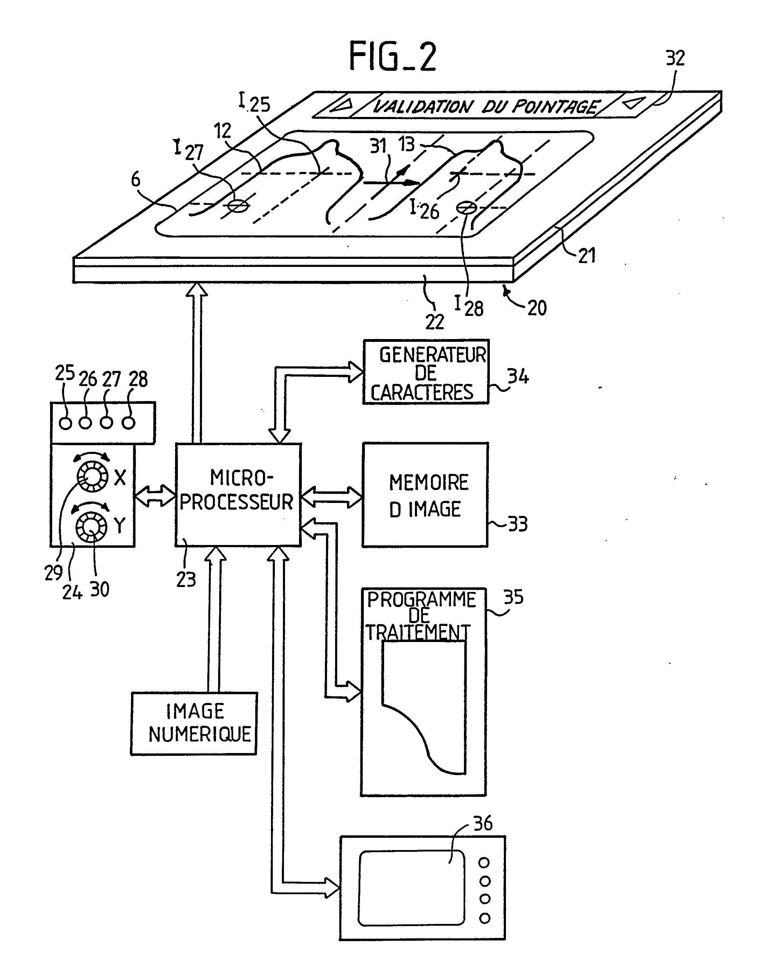

La figure 2 montre la table lumineuse utilisable dans l'invention. La particularité de la table lumineuse de l'invention est de comporter un écran graphique 20 comportant, dans un exemple préféré, une dalle transparente 21 placée au-dessus d'un réseau plan 22 de diodes à électroluminescence. L'écran graphique 20 est commandé par un microprocesseur 23 de telle façon que toutes les diodes du réseau 22 émettent de la lumière. Lorsque l'écran 20 est un écran à plasma, ou encore un écran cathodique, le signal vidéo géré par le microprocesseur est tel que, avant mise en place des index, tous les points de cet écran sont lumineux.

Couplé au microprocesseur, un boîtier de commande 24 permet par un jeu de boutons tel que 25 à 28 de sélectionner un index par exemple $I_{25}$ parmi ceux possibles. Au moyen d'un jeu de potentiomètres permettant des déplacements en X et en Y, 29 et 30, il est possible de déplacer à la surface de l'écran 20 les index $I_{25}$ à $I_{28}$ qui ont été à chaque fois sélectionnés. Le boîtier de commande 24 n'est donné ici qu'à titre indicatif. Il peut notamment comporter d'autres dispositifs de commande plus ergonomiques, notamment une souris ou une boule de poursuite. Lorsqu'on manipule le boîtier de commande 24, on envoie des ordres au microprocesseur pour qu'il déplace les index. Ce faisant, le microprocesseur garde bien entendu en mémoire les adresses (en X et en Y par rapport à un repère 31 placé sur l'écran) de la position de ces index. Il est facile alors, connaissant le pas des éléments d'image de l'écran graphique, de calculer les coordonnés des positions de ces index.

La précision du dispositif de l'invention ne dépend que de la linéarité du pas des éléments d'image. La résolution de la mise en place des index ne dépend que du pas de ces éléments d'image de l'écran graphique lui-même. Ces deux paramètres sont fixés par construction pour la construction d'un écran graphique donné. Etant donné la technologie de production des masques des écrans graphiques, ces positions sont donc très précises et facilement reproductibles d'un écran à un autre. Dans un exemple, avec des écrans de 10 x 20 cm, convenant pour la stéréotaxie en mammographie, il est possible de trouver facilement des écrans dont le pas des éléments d'image est de l'ordre de 0,25 à 0,3 mm. Ceci est suffisant comme précision. On voit immédiatement que le dispositif ainsi réalisé ne peut souffrir d'aucun déréglage. La précision est obtenue par construction et non par un ajustement (souvent répété) d'une tringlerie mécanique. D'autre part, il n'y a pas d'usure et donc très peu d'entretien, d'où une grande fiabilité de l'appareil. Enfin les prix décroissants des composants électroniques permettent de viser des coûts de production nettement inférieurs à ceux actuels.

Mais l'utilisation d'un écran graphique en relation avec un microprocesseur permet d'offrir par ailleurs un plus grand confort d'utilisation. Notamment au moyen d'un générateur de caractères 34, il est possible de différencier les types de couples d'index homologues associés. Par exemple, les index $I_{25}$ et $I_{26}$ sont représentés par une petite croix. A l'opposé, les index $I_{27}$ et $I_{28}$ sont représentés par une petite croix dans un rond. On se rend compte immédiatement que ce choix d'un autre type de caractère permet d'une part d'associer les index entre eux en autant de couples qu'on le désire, et que d'autre part, l'utilisation du microprocesseur associé à l'écran graphique permet de déplacer ces index sans avoir de scrupules quant à un possible chevauchement d'une tringlerie mécanique. Les caractères affichés comme index peuvent être aussi différenciés les uns des autres par la brillance : surbrillance par exemple. Ils peuvent aussi être différenciés par la couleur, si l'écran graphique est un écran couleur. L'index peut donc correspondre à un seul ou à plusieurs éléments d'image (pixels) de l'écran. Dans le même esprit, le générateur de caractères permet, en réservant une région 32 a la périphérie de l'écran 20, d'inscrire des messages, par exemple "VALIDATION DU POINTAGE", qui permettent d'indiquer, à l'opérateur qui manipule le boîtier de commande 24, comment effectuer maintenant l'opération qu'on attend de lui.

En outre, la table lumineuse de l'invention permet toutes les perfectionnements. Par exemple, on peut remplacer la mise en place du film 6 sur la surface de la dalle 21 par l'injection de la représentation numérique de l'image représentée par ce film 6. On sait par ailleurs comment transformer une image de radiologie en une image numérique. Cette image numérique peut alors être stockée, par l'intermédiaire du microprocesseur 23, dans une mémoire d'image 33. Cette mémoire d'image 33 est lue par le microprocesseur 23 et est affichée sur l'écran graphique 20 en même temps et en sur-impression de l'affichage des index $I_{25}$ à $I_{28}$.

Comme on l'a indiqué précédemment, les traitements nécessaires au calcul des coordonnés de la lésion sont connus. Le microprocesseur 23 peut être capable de mettre en oeuvre un tel programme de traitement 35. Il délivre à l'issue une information représentative de la position dans l'espace du point à repérer par l'examen conjugué des deux images stéréographiques. Cette information peut être affichée sur un moniteur de visualisation 36. Cette information peut également être utilisée pour commander le moteur 19 de déplacement de l'aiguille 18 de biopsie.

Pour rendre plus efficace cette chaîne, le film 6 contenu dans le cassette 7 peut être remplacé par un écran intensificateur d'images radiologiques. Le signal vidéo délivré par cet écran intensificateur peut être traité pour être transformé en une image numérique, affichable directement sur l'écran graphique 20. L'opérateur qui a pratiqué l'examen stéréographique peut alors manipuler le boîtier de commande 24 pour indiquer au microprocesseur 3 les positions homologues des points à traiter. Le microprocesseur 23 exécute alors le programme 35 et peut commander, presque en temps réel, le moteur 19. Il peut en résulter une accélération de la phase thérapeutique, toujours pénible pour la patiente.

## Revendications

1. Procédé d'utilisation d'une table (20) lumineuse de radiographie, comportant un écran graphique (21), dans lequel
on illumine un cliché (6) radiographique placé sur cette surface,
on déplace sous le contrôle du microprocesseur au moins un index ($I_{25}$) mobile sur cette surface pour venir pointer un endroit particulier du cliché,
et on contrôle par ce microprocesseur (23) l'écran graphique, pour dispenser une lumière éclairante en tous ses points sauf là où on veut pointer l'index, caractérisé
en ce qu'on contrôle les positions d'au moins deux index ($I_{25}$, $I_{26}$) sur l'écran,
et en ce qu'on associe avec le microprocesseur (35) des adresses en mémoire de ces index pour déterminer, à partir d'un cliché stéréographique placé sur cette table, la position dans l'espace d'un endroit particulier d'un corps (3), ce corps ayant été préalablement soumis à un examen stéréographique qui a donné lieu à la prise de ce cliché stéréographique.

2. Table (20) lumineuse de radiographie, comportant un écran graphique (21) contrôlé par un microprocesseur (23), pour illuminer un cliché (6) radiographique placé sur cette surface et pour contrôler au moins un index (I25) mobile qu'on peut déplacer sur cette surface pour venir pointer un endroit particulier du cliché, afin de dispenser une lumière éclairante en tous ses points sauf là où on veut pointer l'index, caractérisée en ce qu'elle comporte au moins deux index (I25, I26) dont les positions sur l'écran sont contrôlées par le microprocesseur, et en ce que le microprocesseur comporte des moyens (35) d'associer des adresses de ces index pour déterminer, à partir d'un cliché stéréographique placé sur cette table, la position dans l'espace d'un endroit particulier d'un corps (3), ce corps ayant été soumis préalablement à un examen stéréographique qui a donné lieu à la prise de ce cliché stéréographique.

3. Table selon la revendication 2, caractérisée en ce que le microprocesseur peut réserver dans la surface de l'écran graphique, une région (32), de préférence périphérique, pour inscrire des messages en forme de texte.

4. Table selon l'une quelconque des revendications 2 à 3, caractérisée en ce qu'elle comporte en outre une mémoire d'image (33) gérée par le microprocesseur pour afficher directement une version numérique du cliché radiographique à examiner.

5. Table selon l'une quelconque des revendications 2 à 4, caractérisée en ce que l'écran graphique est du type à plasma.

6. Table selon l'une quelconque des revendications 2 à 4, caractérisée en ce que l'écran graphique est du type écran à cristaux liquides.

7. Table selon l'une quelconque des revendications 2 à 4, caractérisée en ce que l'écran graphique est du type écran cathodique.

8. Table selon l'une quelconque des revendications 2 à 4, caractérisée en ce que l'écran graphique est du type écran à matrice de diodes à électroluminescence.

9. Table selon l'une quelconque des revendications 2 à 8, caractérisée en ce que le microprocesseur comporte un générateur (34) de caractères pour afficher plusieurs index de forme et/ou de brillance différentes.

FIG_1

# FIG_2

VALIDATION DU POINTAGE

GENERATEUR DE CARACTERES 34

MICRO-PROCESSEUR

MEMOIRE D IMAGE 33

PROGRAMME DE TRAITEMENT 35

IMAGE NUMERIQUE

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| A | FR-A-2559923 (COMMISSARIAT A L'ENERGIE ATOMIQUE)<br>* abrégé *<br>* page 1, ligne 1 - page 2, ligne 7 *<br>* page 2, ligne 24 - page 3, ligne 25 *<br>* page 4, lignes 4 - 15 *<br>* page 4, ligne 30 - page 5, ligne 8 *<br>* page 6, lignes 8 - 34 *<br>* page 7, ligne 27 - page 8, ligne 20;<br>revendications 1-4; figure 1 * | 1-9 | G03B21/132<br>G03B42/02<br>F21K7/00 |
| A | GB-A-2115544 (D.G.SWINGLER)<br>* figures 1, 2, 5 *<br>* page 1, ligne 61 - page 2, ligne 7 *<br>* page 3, lignes 90 - 96; revendications 1, 5, 6, 8-12 * | 1-9 | |
| A | EP-A-272886 (PFU LTD.)<br>* abrégé *<br>* page 2, lignes 14 - 52 *<br>* page 8, lignes 30 - 44; revendications 1-6, 16, 24; figures 1-4, 7a-7c, 18 *<br>* figure 20 * | 1-4, 9 | |
| A | FR-A-2248535 (JAPAN DIMENSION COMPANY LTD.)<br>* page 2, ligne 23 - page 3, ligne 38 *<br>* page 4, ligne 28 - page 5, ligne 17;<br>revendications 1-8; figures 1-8 * | 1-4 | **DOMAINES TECHNIQUES RECHERCHES (Int. Cl.5)**<br>G02B<br>G03B<br>G06F<br>F21K |
| A | PATENT ABSTRACTS OF JAPAN<br>vol. 12, no. 334 (P-756)(3181) 08 septembre 1988,<br>& JP-A-63 96645 (STANLEY ELECTRIC CO. LTD.) 27 avril 1988,<br>* le document en entier * | 1-9 | |

Le présent rapport a été établi pour toutes les revendications

| Lieu de la recherche | Date d'achèvement de la recherche | Examinateur |
|---|---|---|
| BERLIN | 29 MAI 1990 | MANNTZ, W |